# EUROPEAN PATENT SPECIFICATION

(11) **EP 1 871 902 B1**
(45) Date of publication and mention of the grant of the patent: **27.10.2010**
(21) Application number: 06726800.3
(22) Date of filing: 18.04.2006
(51) Int. Cl.: C12Q 1/68

(54) **METHOD AND DEVICE FOR NUCLEIC ACID SEQUENCING USING A PLANAR WAVE GUIDE**
VERFAHREN UND VORRICHTUNG ZUR NUKLEINSÄURESEQUENZIERUNG UNTER VERWENDUNG EINES PLANAREN WELLENLEITERS
PROCEDE ET APPAREIL POUR LE SEQUENCAGE D'ACIDES NUCLEIQUES UTILISANT UN GUIDE D'ONDES PLANAIRE

(30) Priority: 18.04.2005 GB 0507835
(43) Date of publication of application: 02.01.2008
(73) Proprietor: Illumina Cambridge Limited, Little Chesterford Saffron Walden Essex CB10 1XL (GB)
(72) Inventor: OBRADOVIC, Bojan, Saffron Walden, Essex CB10 1XL (GB); WEST, John, Stephen, Saffron Walden, Essex CB10 1XL (GB); SWERDLOW, Harold, Philip, Saffron Walden, Essex CB10 1XL (GB)
(74) Representative: White, Nina Louise
(86) International application number: PCT/GB2006/001405
(87) International publication number: WO 2006/111729

(56) References cited:
- EP-A- 0 341 928
- WO-A-01/79821
- WO-A-99/47705
- WO-A-03/062791
- US-A1- 2005 153 320
- TOLLEY SAMUEL E ET AL: "Single-chain polymorphism analysis in long QT syndrome using planar waveguide fluorescent biosensors." ANALYTICAL BIOCHEMISTRY, vol. 315, no. 2, 15 April 2003 (2003-04-15), pages 223-237, XP002389412 ISSN: 0003-2697
- DUVENECK GERT L ET AL: "Planar waveguides for ultra-high sensitivity of the analysis of nucleic acids" ANALYTICA CHIMICA ACTA, vol. 469, no. 1, 26 September 2002 (2002-09-26), pages 49-61, XP002389413 ISSN: 0003-2670
- TAITT C R ET AL: "Evanescent wave fluorescence biosensors" BIOSENSORS & BIOELECTRONICS, ELSEVIER SCIENCE PUBLISHERS, BARKING, GB, vol. 20, no. 12, 8 December 2004 (2004-12-08), pages 2470-2487, XP004861161 ISSN: 0956-5663
- VOROS J ET AL: "Optical grating coupler biosensors" BIOMATERIALS, ELSEVIER SCIENCE PUBLISHERS BV., BARKING, GB, vol. 23, no. 17, September 2002 (2002-09), pages 3699-3710, XP004361332 ISSN: 0142-9612
- BIACORE: "Observing DNA at work with Biacore's SPR technology" BIACORE APPLICATION NOTE, no. 5, February 2002 (2002-02),

## Description

### Field of the invention

The present invention is concerned with improvements to methods of imaging nucleotides incorporated into polynucleotides and in particular with improved methods of determining the sequence of template nucleic acid molecules using multiple cycle nucleic acid "sequencing-by-synthesis" reactions.

### Background to the invention

There are known in the art methods of nucleic acid sequencing based on successive cycles of incorporation of fluorescently labelled nucleic acid analogues. In such "sequencing by synthesis" or "cycle sequencing" methods the identity of the added base is determined after each nucleotide addition by detecting the fluorescent label.

In particular, US 5,302,509 describes a method for sequencing a polynucleotide template which involves performing multiple extension reactions using a DNA polymerase or DNA ligase to successively incorporate labelled polynucleotides complementary to a template strand. In such a "sequencing by synthesis" reaction a new polynucleotide strand based-paired to the template strand is built up in the 5' to 3' direction by successive incorporation of individual nucleotides complementary to the template strand. The substrate nucleoside triphosphates used in the sequencing reaction are labelled at the 3' position with different 3' labels, permitting determination of the identity of the incorporated nucleotide as successive nucleotides are added.

Methods of sequencing multiple nucleic acid molecules may be based on the use of arrays, wherein multiple template molecules immobilised on the array are sequenced in parallel. Such arrays may be single molecule arrays or clustered arrays.

Sequencing-by-synthesis reactions generally require imaging or detection steps in which the nature (i.e. identity of the base) of the nucleotides incorporated during the sequencing reaction is determined. Typically the nature of the added base will be determined after each successive nucleotide incorporation step.

Methods for detecting fluorescently labelled nucleotides generally require use of incident light (e.g. laser light) of a wavelength specific for the fluorescent label, to excite the fluorophore. Fluorescent light emitted from the fluorophore may then be detected at the appropriate wavelength using a suitable detection system, such as for example a Charge-Coupled-Device (CCD) camera, which can optionally be coupled to a magnifying device, a fluorescent imager or a microscope. If sequencing is carried out on an array, detection of an incorporated base may be carried out by using a scanning microscope to scan the surface of the array with a laser in order to image fluorescent labels attached to the incorporated nucleotide(s). Alternatively, a sensitive 2-D detector, such as a charge-coupled detector (CCD), can be used to visualise the signals generated. Other techniques such as scanning near-field optical microscopy (SNOM) are available and may be used when imaging dense arrays.

Improvements in imaging/detection technology are a desirable goal in the context of nucleic acid sequencing, particularly when sequencing is carried out on arrays, as this may permit better resolution, allowing more templates to be sequenced in parallel, and improving accuracy, thus enabling longer sequence reads to be obtained from each template.

US Patent No. 5,822,472 describes a process for determining evanescently excited luminescence with a planar dielectric optical sensor platform comprising a transparent substrate to which a planar waveguiding layer is applied. A liquid sample is brought into contact with the waveguiding layer as a superstrate and excitation light coupled into the waveguiding layer produces an evanescent field at the interface between the waveguiding layer and the superstrate. Luminescence produced by substances having luminescent properties either in the liquid sample or immobilised on the waveguiding layer is then measured. This process can be used to detect fluorescent labels attached to biological molecules, particularly in the context of biological affinity assays.

US Patent No. 5,959,292 also describes a process for determining evanescently excited luminescence with a planar dielectric optical sensor platform comprising a transparent substrate to which a planar waveguiding layer is applied. Again the process may be used to detect luminescent substances (such as biological molecules bearing a fluorescent label) present in a liquid sample applied to the waveguiding layer or immobilised on the waveguiding layer. The process may be used to detect hybridisation between a fluorescently labelled target nucleotide present in a liquid sample applied to the waveguiding layer and a complementary capture probe immobilised on the surface of the waveguiding layer.

WO 03/062791 describes a method of detecting single nucleotide polymorphisms in a gene of interest. A plurality of polynucleotide probes are immobilised on a planar waveguide. A fluorescently-labelled analyte is flowed over the planar waveguide. Binding between the labelled analyte and each of the probes causes a change in the fluorescent signal. SNPs are detected by comparing the hybridisation kinetics of the analyte with each of the probes. In this context the use of a planar waveguide is advantageous as it permits detection of fluorescence in real-time, thus allowing comparison of hybridisation kinetics for multiple analyte-probe binding events.

Tolley et al, Analytical Biochemistry, Vol. 315, 223-237 (2003) describes the application of planar waveguide fluorescent biosensor technology in the detection of single nucleotide polymorphisms using a hybridisation assay based on a "capture oligo" immobilised on the waveguide. This biosensor device allows real-time measurement of DNA hybridisation kinetics.

Duveneck et al, Analytica Chimica ACTA, Vol. 469, 49-61 (2002) describes the implementation of genomic microarrays on planar waveguides platforms. The planar waveguide array device platform is used for high throughput analysis of hybridisation assays.

WO99/47705 describes a method of detecting a target polynucleotide using a solution phase nucleic acid sandwich assay, wherein the solid support is an optical planar waveguide, and specific binding is assessed by measuring the luminescence from a label excited in the evanescent field of the waveguide.

Taitt et al, Biosensors and Bioelectronics, Vol. 20, 2470-2487 (2005) is a review of the field of evanescent wave fluorescence biosensors.

Vörös et al, Biomaterials, Vol. 23, 3699-3710 (2002) describes optical grating coupler biosensors and the theory underlying the technique of optical waveguide light mode spectroscopy. Certain applications in the field of protein-DNA interactions are discussed.

WO01/79821 describes an embodiment of a grid-waveguide structure based on a planar thin-layered waveguide.

US 2005/0153320 describes the use of planar waveguides as platforms for single base extension, enabling rapid, real time detection of genetic polymorphisms.

The present inventors have now applied planar waveguide technology to the problem of imaging sequencing-by-synthesis reactions carried out on polynucleotide arrays and have found that the use of a planar wave guide provides important technical advantages. In particular, use of the planar waveguide results in substantial improvements in the sensitivity of detection, meaning in turn that it is possible to accurately score the nature of the incorporated nucleotide over a greater number of cycles of incorporation, resulting in a longer read length. Longer read lengths are important in de novo genome sequencing applications where the aim is to obtain as much accurate sequence information as is technically feasible from a given set of sequencing templates.

In addition, use of a planar wave guide in array-based sequencing methods leads to a substantial reduction in the costs of the associated optical and imaging apparatus, since it is possible to use illuminating laser light of much lower power than is required for prior art methods. Several orders of magnitude enhancement of sensitivity is quite feasible. With the use of lower power lasers a wider range of wavelengths of light become available for excitation purposes, which may in turn expand the range of fluorophores available for use in sequencing.

### Summary of the invention

The invention relates to use of a planar wave guide to enhance the sensitivity of detection of a nucleotide incorporated into a polynucleotide molecule, wherein the incorporated nucleotide is detected by detecting a signal produced by said nucleotide when exposed to an evanescent field generated by coupling of light into said planar waveguide.

In particular, the invention provides a method of determining the identity of the base present in a nucleotide incorporated into a nucleic acid strand complementary to a template nucleic acid immobilised on a planar wave guide comprising:
incorporating a nucleotide into a strand of nucleic acid complementary to the template nucleic acid and determining the identity of the base present in the incorporated nucleotide by detecting a signal produced by said nucleotide when exposed to an evanescent field generated by coupling of light into said planar waveguide.

In preferred embodiments of the invention the incorporated nucleotide is fluorescently labelled.

The method of the invention is useful in many technical applications requiring incorporation of a nucleotide, and particularly a fluorescently labeled nucleotide, into a polynucleotide, including but not limited to sequencing reactions, polynucleotide synthesis, nucleic acid amplification, single nucleotide polymorphism studies, and other such techniques.

The method finds particular application in methods of nucleic acid sequencing.

Therefore, in one aspect the invention provides a method of sequencing a template nucleic acid molecule comprising:
immobilizing said template nucleic acid molecule on a planar waveguide;
incorporating one or more nucleotides into a strand of nucleic acid complementary to the template nucleic acid and determining the identity of the base present in one or more incorporated nucleotide(s) in order to determine the sequence of the template nucleic acid molecule;
wherein the identity of the base present in said nucleotide(s) is determined by detecting a signal produced by said nucleotide(s) when exposed to an evanescent field generated by coupling of light into said planar waveguide.

In a further aspect the invention provides a device comprising a clustered array of template nucleic acid molecules immobilised on a surface of a planar wave guide.

In one embodiment the invention provides a device comprising a clustered array of template nucleic acid molecules immobilised on the surface of a planar wave guide,
a top plate held in liquid seal against at least a portion of the surface of the planar wave guide on which the template nucleic acid molecules are immobilised to form a flow cell enclosing at least one reaction channel,
an inlet port and an outlet port to permit flow of liquid through the reaction channel when the device is in use, and
an input coupling grating.

In one embodiment the device may further include an output coupling grating, wherein the input and output coupling gratings are positioned on either side of a reaction channel.

In a preferred embodiment the device may comprise two or more reaction channels, wherein a coupling grating is positioned between each adjacent pair of reaction channels

### Brief description of the drawings

Figure 1(a) shows a cross section through a device suitable for use in the process of the invention, Figure 1(b) is a plan view of the same device.
Figure 2 is a schematic illustration of the device of Figure 1 when in use, in perspective view, illustrating the path of light through the planar wave guide and the optical configuration of illumination and detection.
Figure 3 shows a cross section through a further device suitable for use in the process of the invention.
Figure 4 is a schematic illustration of a device similar to that illustrated in Figure 3 when in use, illustrating the path of light through a portion of the wave guide and the optical configuration for illumination and detection.
Figure 5 illustrates an alternative optical configuration.
Figure 6 is a schematic illustration of optical instrumentation for use in the method of the invention.
Figure 7 illustrates the sequence of a DNA molecule comprising a lambdaF fragment which can be used in the construction of a clustered array of template polynucleotides for sequencing using solid-phase nucleic acid amplification. The sequences and primer binding sites for the primers P7, P5 and #562 are also shown.

### Detailed description of the invention

The invention will now be further described. In the following passages different features of the invention are defined in more detail. Each feature so defined may be combined with any other feature or features unless clearly indicated to the contrary. In particular any feature indicated as being preferred or advantageous may be combined with any other feature or features.

In one important aspect the invention relates to a method of "sequencing-by-synthesis" which is carried out on a planar waveguide.

In the context of this invention the terms "sequencing reaction", "sequencing methodology" or "method of sequencing" generally refer to any polynucleotide "sequencing-by-synthesis" reaction which involves sequential addition of one or more nucleotides to a growing polynucleotide chain in the 5' to 3' direction using a polymerase in order to form an extended polynucleotide chain complementary to the template nucleic acid to be sequenced. The identity of the base present in one or more of the added nucleotide(s) is determined in a detection or "imaging" step. The identity of the added base is preferably determined after each nucleotide incorporation step. The sequence of the template may then be inferred using conventional Watson-Crick base-pairing rules. For the avoidance of doubt "sequencing" can also encompass incorporation and identification of a single nucleotide. Determination of the identity of a single base may be useful, for example, in the scoring of single nucleotide polymorphisms.

The identity of the base present in one or more nucleotides incorporated during the sequencing reaction is determined by detecting a signal produced when the nucleotide(s) are exposed to evanescent and evanescent field generated when incident light is coupled into the planar wave guide. In order to generate such a signal the incorporated nucleotides may comprise a label moiety. Suitable labels include any substance which produces a detectable signal on exposure evanescent illumination. Preferred label moieties are luminescent substances.

The preferred label is a fluorophore, which, after absorption of energy, emits radiation at a defined wavelength. Many suitable fluorescent labels are known.
For example, Welch et al. (Chem. Eur. J. 5(3):951-960, 1999) discloses dansyl-functionalised fluorescent moieties that can be used in the present invention. Zhu et al. (Cytometry 28:206-211, 1997) describes the use of the fluorescent labels Cy3 and Cy5, which can also be used in the present invention. Labels suitable for use are also disclosed in Prober et al. (Science 238:336-341, 1987); Connell et al. (BioTechniques 5(4):342-384, 1987), Ansorge et al. (Nucl. Acids Res. 15(11):4593-4602, 1987) and Smith et al. (Nature 321:674, 1986). Other commercially available fluorescent labels include, but are not limited to, fluorescein, rhodamine (including TMR, Texas red and Rox), alexa, bodipy, acridine, coumarin, pyrene, benzanthracene and the cyanins.

For example, two classes of particularly preferred fluorophores which may be used according to this invention are the Alexa series of Molecular Probes, (sometimes referred to as Alexa Fluor dyes) and fluorescent labels in the Atto series available from Atto-tec (sometimes referred to as Atto fluorescent labels) of Atto-tec.

Multiple labels can also be used in the invention. For example, bi-fluorophore FRET cassettes (Tet. Letts. 46:8867-8871, 2000) are well known in the art and can be utilised in the present invention. Multi-fluor dendrimeric systems (J. Amber. Chem. Soc. 123:8101-8108, 2001) can also be used.

Although fluorescent labels are preferred, other forms of detectable labels will be apparent as useful to those of ordinary skill. For example, microparticles, including quantum dots (Empodocles, et al., Nature 399:126-130, 1999), gold nanoparticles (Reichert et al., Anal. Chem. 72:6025-6029, 2000).

Multi-component labels can also be used in the invention. A multi-component label is one which is dependent on the interaction with a further compound for detection. The most common multi-component label used in biology is the biotin-streptavidin system. Biotin is used as the label attached to the nucleotide base. Streptavidin is then added separately to enable detection to occur. Other multi-component systems are available. For example, dinitrophenol has a commercially available fluorescent antibody that can be used for detection.

The label (or label and linker construct) can be of a size or structure sufficient to act as a block to the incorporation of a further nucleotide. This permits controlled polymerization to be carried out. The block can be due to steric hindrance, or can be due to a combination of size, charge and structure.

The labels may be the same for each type of nucleotide, or each nucleotide type may carry a different label. This facilitates the identification of incorporation of a particular nucleotide. Thus, for example modified adenine, guanine, cytosine and thymine may each have attached a different fluorophore to allow them to be discriminated from one another readily. When sequencing on arrays, a mixture of labelled and unlabelled nucleotides may be used.

Detectable labels such as fluorophores can be linked to nucleotides via the base using a suitable linker. The linker may be acid labile, photolabile or contain a disulfide linkage. Preferred labels and linkages include those disclosed in WO03/048387. Other linkages, in particular phosphine-cleavable azide-containing linkers, may be employed in the invention as described in greater detail in WO2004/018493.

The nucleotides described in W02004/018493 comprise a purine or pyrimidine base and a ribose or deoxyribose sugar moiety which has a removable blocking group covalently attached thereto, preferably at the 3 'O position. 3' blocking groups are also described in WO2004/018497. Use of such 3'-blocked nucleotides permits controlled incorporation of nucleotides in a stepwise manner, since the presence of a blocking group at the 3'-OH position prevents incorporation of additional nucleotides. The detectable label may, if desirable, be incorporated into the blocking groups as is disclosed in W02004/018497.

The method of the invention may comprise determination of the identity of the incorporated base over at least one, cycle of nucleotide incorporation. For the avoidance of doubt the term "sequencing" encompasses incorporation and identification of a single nucleotide. Determination of the identity of a single base may be useful, for example, in the scoring of single nucleotide polymorphisms. In other embodiments the identity of at least two, preferably at least 10, and more preferably at least 16 incorporated nucleotides may be determined.

The ability to accurately sequence 10 or more, and preferably 16 or more, consecutive nucleotides in a sequencing reaction is a significant advantage in applications such as human genome re-alignment and identification and/or scoring of single nucleotide polymorphisms in the human genome. In applications involving human genome re-sequencing or scoring/identification of SNPs in the human genome it is preferred that from 16 to 30 nucleotides are successively incorporated, and identified, in the sequencing reaction.

The method is not, however, intended to be limited to sequencing reads of up to 30 nucleotides. For *de novo* genome sequencing applications it is desirable to maximise the sequencing read length. As aforesaid, a particular advantage of the use of planar wave guide technology in sequencing-by-synthesis is that it can enhance sensitivity, improving the accuracy of base calling over longer read lengths, meaning that accurate sequence reads of the order of 100 nucleotides are achievable.

The features of planar wave guides are generally known in the art, and described for example in US Patent Nos. 4,582,809, 5,822,472 and 5,959,292 and International patent application Nos. WO 90/06503 and WO 03/062791. Planar waveguides typically comprise an optically transparent substrate to one surface of which is applied a waveguiding layer formed of an optically transparent material having a refractive index greater than that of the substrate. Suitable materials for construction of the substrate part of the planar waveguide include, but are not limited to, glass, quartz or optical plastic. Suitable materials to form the waveguide layer include inorganic substances such as metal oxides, for example TiO₂, ZnO, Nb₅O₅, Ta₂O₅, HfO₂ or ZrO₂. Ta₂O₅ and TiO₂ are particularly preferred.

Template nucleic acid molecules to be sequenced using the method of the invention are either immobilised on the external surface of the waveguiding layer, or held in close proximity to the waveguiding layer.

The term "immobilised" encompasses direct or indirect attachment of the template nucleic acid to the external surface of the waveguiding layer. Templates must be held in sufficiently close proximity to the surface of the waveguide to enable nucleotides incorporated into the complementary strand formed during the sequencing reaction to be illuminated by the evanescent field generated by coupling of light into the waveguide, but it is generally not essential for the templates to be directly attached to the material of the waveguiding layer. Indeed, the templates need not be attached to the planar wave guide at all, provided that they are held in sufficiently close proximity to the wave guide to be penetrated by the evanescent field. The templates may be immobilised on some other component of a device incorporating the wave guide, such as the top plate of the device described below with reference to the drawings.

Direct attachment to the material of the waveguiding layer may be accomplished, for example, by hydrophobic absorption or covalent bonding.

In other embodiments the surface of the waveguiding layer may be functionalised by addition of an intermediate layer making it suitable for attachment of polynucleotides, provided that this does not unduly affect production of the evanescent field or prevent the evanescent field from penetrating beyond the "functionalised" surface of the waveguide. For example, the surface of the waveguide can be functionalised by silanisation or by applying a polymer layer.

A particularly preferred group of molecules with which the waveguide layer may be treated are silanes of the general formula RnSiX(4-n) (wherein R is an inert moiety that is displayed on the surface of the solid support, n is an integer from 1-4 and X is or comprises a reactive leaving group, such as a halide (e.g. Cl, Br) or alkoxide (e.g. X may be a C1-6 alkoxide). An example of such an X moiety which comprises a reactive leaving group is haloacetamido alkyl (e.g. of formula (CH₂)ₘ N(H)C(O)Y, wherein m is an integer of from 1 to 20, preferably 3 to 10, and Y is a halogen such as bromine, chlorine or iodine, preferably bromine). Particularly preferred silanes for use in this invention, so as to produce appropriately modified surfaces, include silanes such as tetraethoxysilane, triethoxymethylsilane, diethoxydimethylsilane, glycidoxypropyltriethoxysilane, or triethoxybromoacetamidopropyl silane although many other suitable examples will be apparent to the skilled person.

Preferably mixtures of silanes are employed. Mixtures of tetraethoxysilane and triethoxybromoacetamidopropyl silane (e.g. in a ratio of 1:100) are particularly preferred. Such silane-functionalised surfaces permit attachment of nucleic acid molecules having a thiophosphate or phosphorothioate functionality.

The nucleic acid template to be sequenced in a sequencing reaction may be any polynucleotide that it is desired to sequence. The nucleic acid template for a sequencing reaction will typically comprise a doublestranded region having a free 3' hydroxyl group which serves as a primer or initiation point for the addition of further nucleotides in the sequencing reaction. The region of the template to be sequenced will overhang this free 3' hydroxyl group on the complementary strand. The primer bearing the free 3' hydroxyl group may be added as a separate component (e.g. a short oligonucleotide) which hybridises to a region of the template to be sequenced. Alternatively, the primer and the template strand to be sequenced may each form part of a partially self-complementary nucleic acid strand capable of forming an intramolecular duplex, such as for example a hairpin loop structure. Nucleotides are added successively to the free 3' hydroxyl group, resulting in synthesis of a polynucleotide chain in the 5' to 3' direction. After each nucleotide addition the nature of the base which has been added may be determined, thus providing sequence information for the nucleic acid template.

The term "incorporation" of a nucleotide into a nucleic acid strand (or polynucleotide) refers to joining of the nucleotide to the free 3' hydroxyl group of the nucleic acid strand via formation of a phosphodiester linkage with the 5' phosphate group of the nucleotide. Preferably the nucleotide will be incorporated into the polynucleotide in an enzymatic reaction catalysed by an enzyme with polymerase activity.

The nucleic acid template to be sequenced may be DNA or RNA, or even a hybrid molecule comprised of deoxynucleotides and ribonucleotides. The nucleic acid may comprise naturally occurring and/or non-naturally occurring nucleotides and natural or non-natural backbone linkages.

If the templates to be sequenced are immobilised on a planar wave guide to form an "array" then the array may take any convenient form. Thus, the method of the invention is applicable to all types of "high density" arrays, including single-molecule arrays and clustered arrays.

The method of the invention may be used for sequencing on essentially any type of array formed by immobilisation of nucleic acid molecules on a planar wave guide, and more particularly any type of high-density array. However, the method of the invention is particularly advantageous in the context of sequencing on clustered arrays.

In multi-polynucleotide or clustered arrays distinct regions on the array comprise multiple polynucleotide template molecules. Depending on how the array is formed each site on the array may comprise multiple copies of one individual polynucleotide molecule or even multiple copies of a small number of different polynucleotide molecules (e.g. multiple copies of two complementary nucleic acid strands).

Multi-polynucleotide or clustered arrays formed from immobilised nucleic acid strands may be produced using techniques generally known in the art. By way of example, WO 98/44151 and WO 00/18957 both describe methods of nucleic acid amplification which allow amplification products to be immobilised on a solid support in order to form arrays comprised of clusters or "colonies" of immobilised nucleic acid strands. Analogous amplification techniques can be used to synthesise clustered arrays on solid supports incorporating planar wave guides.

WO 98/44152 and WO 00/18957 both describe methods of parallel sequencing of multiple templates located at distinct locations on a solid support, and in particular sequencing of arrays of nucleic acid colonies. The methods described therein, and indeed any other known method of sequencing nucleic acid clusters, may be used to sequence clustered arrays formed on a solid supports incorporating planar wave guides in accordance with the invention.

The method of the invention may also be used in the context of sequencing on "single molecule arrays" formed on solid supports incorporating a planar wave guide.

Single molecule arrays are generally formed by immobilisation of a single polynucleotide molecule at each discrete site that is detectable on the array. Single-molecule arrays comprised of nucleic acid molecules that are individually resolvable by optical means and the use of such arrays in sequencing are described, for example, in WO 00/06770. Single molecule arrays comprised of individually resolvable nucleic acid molecules including a hairpin loop structure are described in WO 01/57248. Single molecule arrays can be formed on substrates incorporating a planar wave guide using techniques similar to those described in WO 00/06770 or WO 01/57248.

In order to carry out the sequencing reaction the surface of the planar wave guide on which the template nucleic acids are immobilised must be brought into contact with liquid reagents required for the sequencing reaction. Advantageously this may be accomplished by forming a flow cell which permits controlled flow of reagents over the surface of the planar wave guide. Construction and operation of such flow cells is described in further detail hereinbelow, with reference to the drawings.

A sequencing reaction requires the following components to interact under conditions which permit the formation of a phosphodiester linkage between the 5' phosphate group of a free nucleotide to be incorporated and a free 3' hydroxyl group on the DNA template:
a template polynucleotide with suitable primer (providing free 3' -OH group required for addition of a further nucleotide);
a nucleotide solution comprising at least one suitably labelled nucleotide; and
a polymerase capable of catalysing incorporation of labelled nucleotides into a polynucleotide.

Suitable polymerase enzymes which efficiently incorporate nucleotide analogues including large substituents at the 3' hydroxyl position are described in WO 2005/024010.

The structure and construction of a device for use in the process of the invention will now be further described, by way of example, with reference to the accompanying Figures.

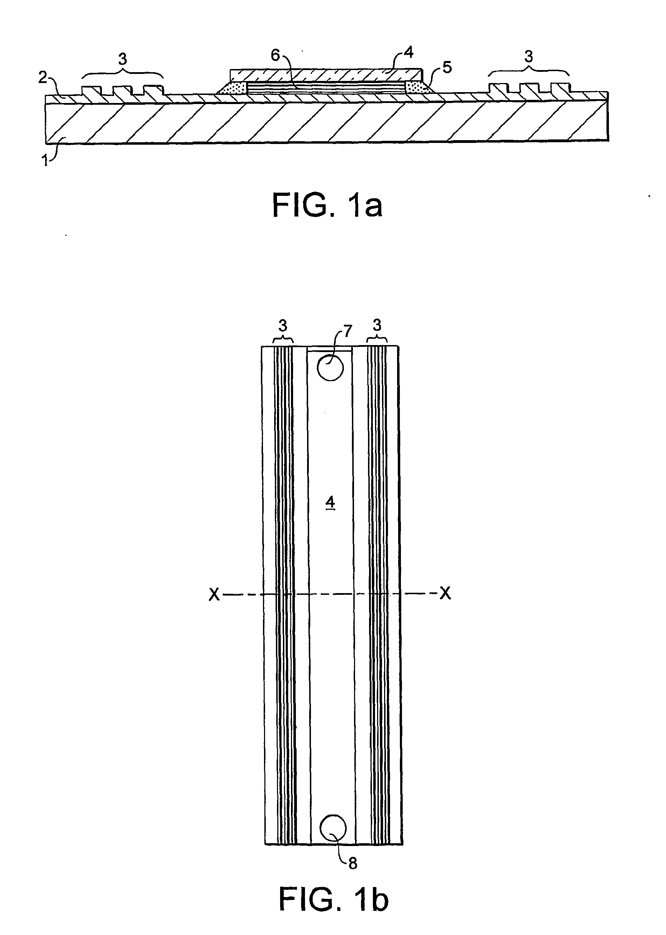

Figure 1 shows a cross-section through a representative device suitable for use in the process of the invention. The device includes a planar wave guide comprised of a substrate 1 formed of an optically transparent material. A waveguide layer 2 formed of an optically transparent material of higher refractive index than the substrate material is applied to one planar surface of the substrate. An input coupling grating 3 and output coupling grating 3' are formed in the waveguide layer. The input grating is required to couple light into the wave guide and is therefore an essential feature. The output coupling grating is not essential but can be advantageous in certain embodiments of the invention (as discussed further below). The output coupling grating provides a means to measure the efficiency of the input coupling grating, and thus optimise the optical coupling of light onto the sample.

In the illustrated embodiment the input and output gratings are positioned on either side of the reaction channel 6 in order to guide illuminating light through a portion of the waveguide which forms the bottom surface of the flow cell. The gratings may be produced in the wave guide layer itself or gratings may be etched into the substrate during manufacture and a waveguide layer applied thereto, such that the grating pattern is transferred to the waveguide layer. The dimensions and construction of input and output coupling gratings are generally known in the art.

A top plate 4 is sealed against the surface of the planar wave guide bearing the waveguide layer by a liquid seal 5 in order to form a flow cell enclosing a reaction channel 6 through which liquid reagents can flow when the device is in use. Use of the term "channels" is not intended to imply any particular shape or configuration. The reaction channel is simply an enclosed volume through which liquid reagents can flow when the device is in use. The chosen dimensions of the reaction channel will generally be determined by the field of view of the imaging instrumentation, as discussed below.

If the device is to be used in conjunction with an optical configuration in which detection of the signal produced in the sequencing reaction is carried out through the top plate 4, the latter must be formed of an optically transparent material such as glass or a suitable plastic.
If the optical configuration allows detection through the substrate surface of the waveguide then the top plate need not be transparent and the material can be selected to have advantageous physical or chemical properties. For example, the material of the top plate may be selected to allow a particular surface attachment strategy/chemistry, enabling template nucleic acid molecules to be immobilised on the inner surface of the top plate (the inner surface being that which faces the interior of the reaction channel when the flow cell is assembled). In other embodiments the material of the top plate may be selected to allow easier heating or cooling of the reaction channel of the flow cell.

The liquid seal 5 may be provided by any suitable liquid sealing means. In one embodiment the sealing means may be an adhesive. For subsequent use in the sequencing process of the invention the adhesive must be stable at temperatures up to 95°C. The adhesive must also have optical properties such that it does not couple light out of the waveguide layer. The adhesive must also be capable of adhering to the material of the waveguide layer, as well as the material of the top plate, in order to provide an effective liquid seal. Suitable adhesives include, but are not limited to, conventional microscope sealants. It is not essential to use an adhesive, other sealing means may be used, such as gaskets clamped to maintain a liquid seal.

The dimensions of the flow cell, and more specifically of the reaction channel enclosed therein, will generally be determined by the field of view of the imaging system to be used in conjunction with the device. Typically the reaction channel will measure 1mm in its shortest dimension in the plane parallel to the surface of the waveguide.

Figure 1(b) shows a schematic plan view of the same device in which the overall shape of the device is more clearly visible. This particular device is rectangular but this is not essential. The overall shape and external dimensions of the device will generally by determined by the instrumentation used for illumination and detection.

Figure 1(b) illustrates the configuration of the flow cell enclosing a narrow reaction channel 6, which in this particular embodiment is about 1mm wide. An inlet port 7 and outlet port 8 are provided in order to enable controlled flow of reagents through the channel. Flow of reagents through the channel when the device is in use may be regulated with the use of a suitable pump (e.g. a peristaltic pump). In this embodiment the inlet ports and outlet ports are formed in the top plate, at opposite ends of the device, but this is not essential. The inlet and outlet ports could be provided through the waveguide substrate itself or at the interface between the top plate and the wave guide and may take any suitable configuration which provides for liquid flow through the reaction channel enclosed in the flow cell.

Nucleic acid templates to be sequenced using the process of the invention are enclosed within the reaction channel of the flow cell. As described above, the templates may be immobilised on the planar wave guide or held in close proximity to it, for example they may be immobilised on the inner surface of the top plate. If immobilisation of the templates requires coating of the wave guide with an intermediate layer to facilitate indirect attachment of the templates to the surface of the wave guide, this may be carried out before or after assembly of the flow cell. Coating with the intermediate layer may be confined just to the portion of the surface of the wave guide that will be enclosed within the reaction channel. The templates may be immobilised on the surface of the wave guide before or after the flow cell is assembled. The flow cell assembly may advantageously be used to carry out a reaction leading to immobilisation of the template molecules, such as for example a solid-phase amplification reaction.

A heating element (not shown in Figure 1) is used to apply heat to the contents of the reaction channel 6 when the device is in use. Conveniently heat can be applied to the underside of the substrate using a Peltier element or other suitable heat source, which may be a separate component, not integrally formed with the wave guide device itself.

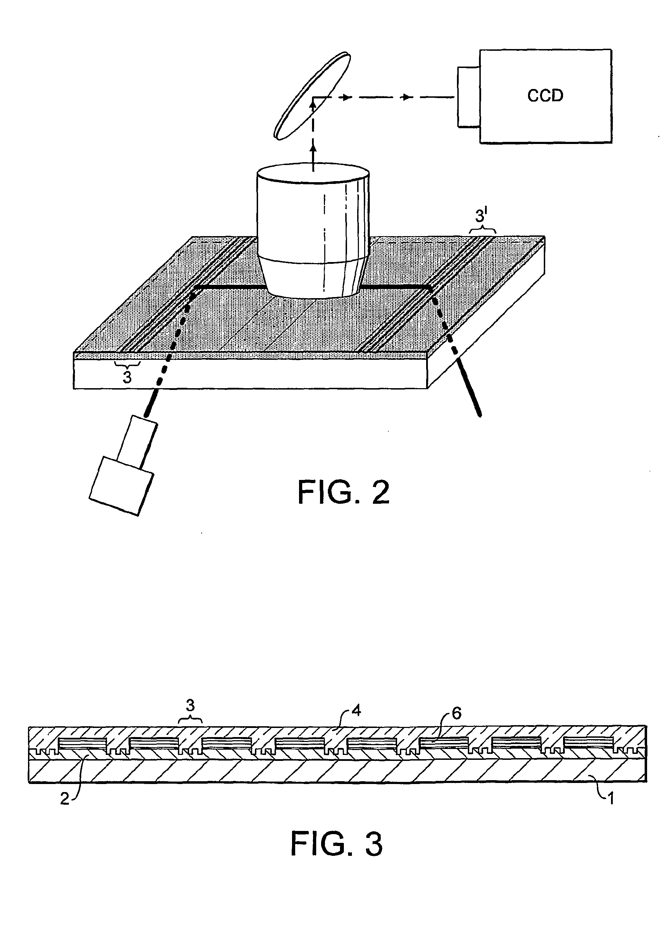

Figure 2 schematically illustrates an optical configuration for use with the device illustrated in Figure 1. Laser light is incident on an input coupling grating 3 and is coupled into the wave guide layer. An output coupling grating 3' couples light out of the wave guide. Any suitable laser source can be used to provide incident light at a wavelength appropriate for excitation of labels used in the sequencing reaction. For standard commercially available fluorescent labels typical wavelengths are in the range of from 300-800 nm, or from 400-800 nm. The illuminated area in this embodiment is approximately 1 mm². Imaging/detection can be carried out using any suitable detection instrument capable of resolving signals from either individual polynucleotide clusters or colonies, in the case of sequencing on a clustered array, or individual polynucleotide molecules, in the case of sequencing on a single molecule array. In the example illustrated in Figure 2 detection is carried out using a fluorescence microscope objective coupled to a charge coupled detector (CCD). The fluorescence objective is positioned on top of the top plate (external to the flow cell), hence it is necessary for the top plate to be optically transparent.

The device illustrated in Figure 1 includes a single flow cell enclosing a single reaction channel with associated input and output coupling gratings. In other embodiments devices can be constructed with a flow cell enclosing multiple (two or more) reaction channels. A device with multiple reaction channels is illustrated schematically in Figure 3. The device again comprises a substrate 1 and a waveguiding layer 2 deposited on a planar surface of the substrate. A top plate 4 is sealed against the surface bearing the waveguide layer to form a flow cell enclosing multiple reaction channels 6 through which liquid reagents may flow. Multiple coupling gratings 3 are present in the wave guide layer, such that a grating is present between each adjacent pair of reaction channels. The dimensions of the channels enclosed in the flow cell will again largely be determined by the field of view of the chosen detection/imaging system. Typically the shortest dimension of each channel will be 1mm in the plane parallel to the wave guide surface.

Each individual reaction channel in the flow cell may be provided with separate inlet and outlet ports and associated fluidics to allow flow of liquid reagents through each of the reaction channels. A multi-channel pump may be used to control reagent flow through each of the reaction channels. The inlet ports to each of the individual reaction channels may be in fluid communication with a single reagent reservoir, for example via a reagent input channel or conduit, thus enabling supply of reagents to each of the reaction channels from a common reagent source. Similarly, the outlet ports from each of the individual flow cells may be in fluid communication with a common outlet channel or conduit. If desired, flow to the individual flow cells may be separately controlled with the use of valves etc. Thus, when the device is in use reagents the required for the sequencing reaction may be simultaneously introduced into each of the reaction channels, meaning that sequencing reactions occur in parallel in each of the reagent channels, or alternatively reagent flow to each individual reaction channel may be separately controlled, for example enabling sequential addition of reagents to individual channels.

Multi-channel devices such as that illustrated in Figure 3 can be manufactured using conventional microfabrication techniques. For example, a glass or optical plastic substrate can be manufactured with recesses corresponding to the reaction channels required in the assembled device. Coupling gratings may also be etched into the substrate at this stage. A wave guide layer is then applied to the surface of the substrate provided with the reaction channel recesses. A top plate, optionally including recesses which correspond to those in the substrate, inlet ports and outlet ports, may then be sealed against the wave guide in order to form the flow cell enclosing multiple reaction channels.

As well as coupling light into the wave guide, the gratings between adjacent pairs of reaction channels can also couple light out of the wave guide. This provides a degree of flexibility in that it is possible to control illumination in the portion of the wave guide associated with each of the reaction channels. Limitations of available detection instrumentation mean that it is generally not possible to provide a field of view which is large enough to image the whole of a sequencing array simultaneously yet still achieve resolution at the level of single clusters or single molecules. Hence, areas of the array are scanned by the detection instrumentation. If the whole of the array is illuminated during the course of the detection procedure it is likely that some areas of the array may become photobleached by the illuminating light before they are scanned by the detection instrument. Hence, there may be a loss of sensitivity over certain areas of the array. This is a particular problem with multiple cycles of nucleotide incorporation and detection. Using a multiple reaction channel device such as that shown in Figure 3 it is possible to control illumination in specific areas of the array and thus reduce or prevent photobleaching in areas of the array that have yet to be been scanned by the detection instrument. The inlet and outlet coupling gratings allow the user to confine illumination to the area it is intended to measure without pre-bleaching other areas of the array prior to imaging. Thus the channel widths are advantageously optimised to be similar to the area of illumination that can be achieved with the optical imaging instrumentation.

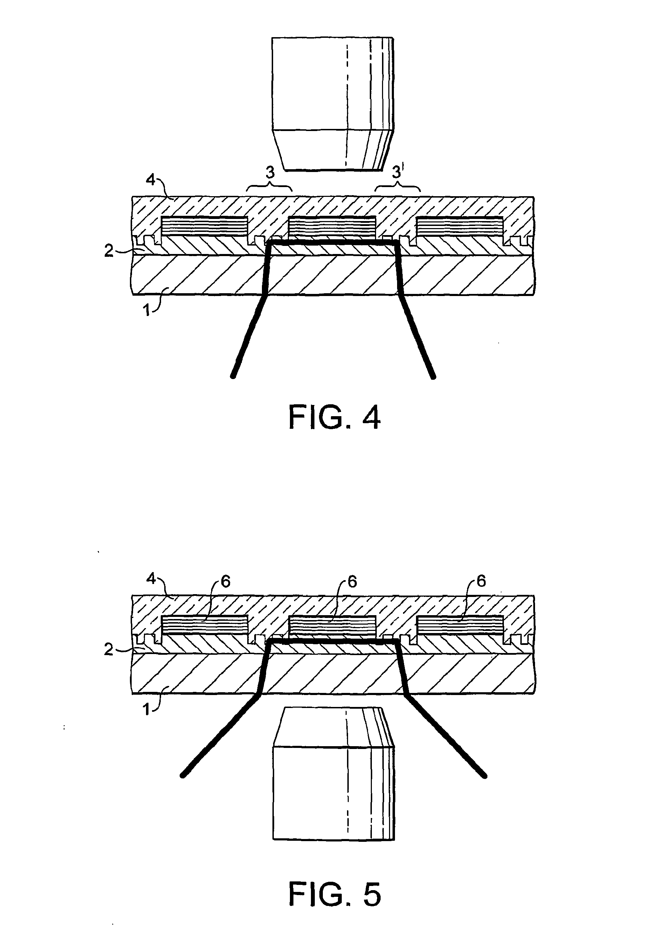

Figure 4 schematically illustrates one optical configuration of illumination and detection which can be used in conjunction with a multiple reaction channel device of the type illustrated in Figure 3. Light incident on an input coupling grating 3 is coupled into the wave guide and is coupled out of the wave guide by output coupling grating 3'. Thus, the path of the incident light is largely confined to the area of the wave guide corresponding to the reaction channel in the field of view of the detection instrument. The detection instrument is again a fluorescence microscope objective linked to a CCD. In this configuration it is essential for both the substrate 1 and the top plate 4 to be formed from an optically transparent material.

Figure 5 illustrates an alternative optical configuration of illumination and detection which can be used in conjunction with a multiple reaction channel device of the type illustrated in Figure 3. By altering parameters of the diffraction grating (groove density, pitch, blazed or sinusoidal gratings) and/or changing the material of the substrate (i.e. modification of the index of refraction) it may be possible to use a greater angle of incidence for light incident on the first grating 3. This enables the detection instrument (e.g. fluorescence microscope objective) to be placed on the same side of the device as the illuminating light source. Consequently, there is no requirement for top plate 4 to be optically transparent. A range of non-transparent materials having advantageous properties may be used, such as materials which allow surface attachment of template nucleic acids or materials which allow easier heating or cooling of the reaction channel 6. In such an embodiment a heating element (e.g. Peltier element) may be placed adjacent to the top plate rather than on the underside of the substrate in order to control the temperature of reagents in the reaction channels or chambers 6.

The following example is intended to be illustrative of, not limiting to, the invention.

### Example

### Construction of a flow cell

Glass slides coated with a Ta₂O₅ wave guide layer are obtained from Uniaxis.

A flow cell is then assembled by gluing a glass cover slip to the surface of the wave guide layer using microscope sealant. The glue forms a liquid seal, such that a portion of the wave guide surface is enclosed within the flow cell. Two holes in the cover slip provide an inlet port and an outlet port. Teflon tubing can be tightly flanged against the inlet and outlet ports to provide fluid connection to an external peristaltic pump.

### Functionalisation of the wave guide

An intermediate layer of functionalised polyacrylamide hydrogel is applied to the surface of the wave guide layer enclosed within the flow cell as follows:

Acrylamide (Purity 99 +%, 0.4 g) is dissolved in MilliQ H₂O (10 mls) (Solution I). Potassium or ammonium persulfate (0.25 g) is dissolved in MilliQ H₂O (5 ml) (Solution II). N-(5- bromoacetamidylpentyl)acrylamide (BRAPA) (33 mg) is dissolved in DMF (330 µl) (Solution III).

Solution I is degassed 10 minutes with argon. Solution III is added to solution I. After mixing, N, N, N',N'-tetramethylethylenediamine (TEMED) (23 µl) is added to the mixture. Finally solution II (200 µl) is added. The polymerisation mixture is rapidly reacted with the planar wave guide slides at room temperature for 1.5 hr. The slides are then washed thoroughly under running MilliQ H₂O, dried and stored under argon.

### Solid-phase amplification

A clustered array of template polynucleotides is prepared on the functionalised surface of the wave guide using solid-phase amplification to produce nucleic acid colonies. The amplification reaction is carried out within the flow cell.

The solid-phase amplification procedure involves an initial step of covalent attachment (grafting) of primers to the functionalised surface of the wave guide (i.e. to the intermediate layer of polyacrylamide hydrogel) to generate a surface ready for use in PCR. The PCR template may be hybridised to attached primers immediately prior to the PCR reaction. The PCR reaction thus begins with an initial primer extension step rather than template denaturation.

5'-phosphorothioate oligonucleotides are grafted onto the surface of the hydrogel in 10 mM phosphate buffer pH7 for 1h at RT. The following exemplary primers are used .
P7 primer with polyT spacer:
   5'phosphorothioate-TTTTTTTTTTCAAGCAGAAGACGGCATACGA -3'
P5 primer with polyT spacer:
   5'phosphorothioate-TTTTTTTTTTAATGATACGGCGACCACCGA -3'

Primers are used at a concentration of 0.5 µM in the grafting solution.

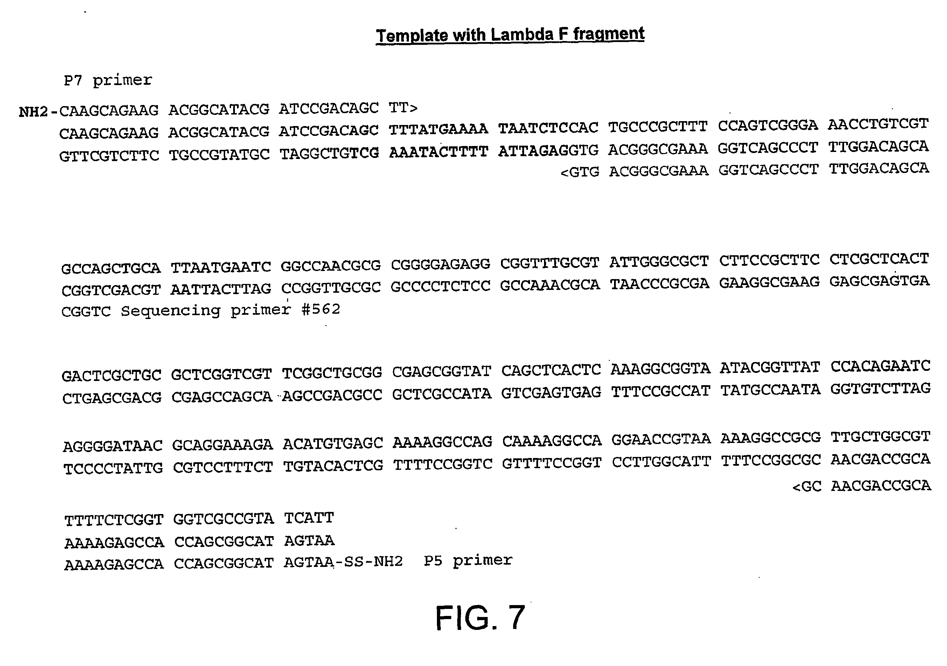

The exemplary template used is a fragment of pBluescript T246 containing primer-binding sequences which enable PCR amplification to be carried out using the P7/P5 primers shown above (see Figure 7). The hybridization procedure begins with a heating step in a stringent buffer (95°C for 5 minutes in TE) to ensure complete denaturation prior to hybridisation of the template. Hybridization is then carried out in 5x SSC, using template diluted to a final concentration of 5 nM. After the hybridization, the chip is washed for 5 minutes with milliQ water to remove salts.

Surface amplification is carried out by thermocycled PCR in an MJ Research thermocycler.

A typical PCR program is as follows:
1- 97.5°C for 0:45
2- X°C for 1:30
3- 73°C for 1:30
4- Goto 1 [40] times
5- 73°C for 5:00
6- 20°C for 3:00
7- End

Since the first step in the amplification reaction is extension of the primers bound to template in the initial hybridisation step the first denaturation and annealing steps of this program are omitted (i.e. the chip is placed on the heating block only when the PCR mix is pumped through the flow cell and the temperature is at 73°C).

The annealing temperature (X°C , step 2) depends on the primer pair that is used. Experiments have determined an optimal annealing temperature of 57°C for P5/P7 primers. For other primer-pairs the optimum annealing temperature can be determined by experiment. The number of PCR cycles may be varied if required.

PCR is carried out in a reaction solution comprising 1x PCR reaction buffer (supplied with the enzyme) 1M betain, 1.3% DMSO, 200 µM dNTPs and 0.025 U/µL Taq polymerase.

General features of the solid-phase amplification procedure to produce nucleic acid colonies are described in International patent application WO 00/18957.

### Template linearisation

Templates to be sequenced may optionally be linearised prior to the sequencing reaction. The linearisation procedure removes all or a substantial part of one strand of each duplex produced by solid-phase PCR from the surface, leaving the complementary strand attached to the surface to provide a sequencing template.

Linearisation can be carried out by enzymatic cleavage with a suitable restriction enzyme that cleaves one strand of each duplex at a corresponding restriction site. A restriction site may be engineered into one of the primers used for the solid-phase amplification to ensure that the required restriction site is present in the amplified strands. A particularly preferred enzyme is BglII, but the invention is not intended to be limited to this specific enzyme.

### Thermal dehybridisation.

Thermal denaturation or de-hybridization of the colonies is carried out in stringent buffer (TE). Temperature is ramped 0.5°C/sec to 97.5°C and held at 97.5°C for 2 minutes 30 seconds.

### Hybridisation of sequencing primer

The procedure begins with a heating step in a stringent buffer (TE) to ensure complete denaturation of the colonies prior to hybridisation of the sequencing primer. Hybridization is carried out in 5x SSC, using an oligonucleotide sequencing primer diluted to a final concentration of 500 nM. A typical temperature cycling profile is as follows:
MJ-Research Thermocycler program set:
   (Control method: block)
      1- 0.5°C/sec to 97.5°C
      2- 97.5°C for 2:30
      3- 97.5°C for 0:02
         -0.1°C per cycle
      4- Goto 3 for 574 times
      5- 40°C for 15:00
      6- End

### Long read sequencing protocol

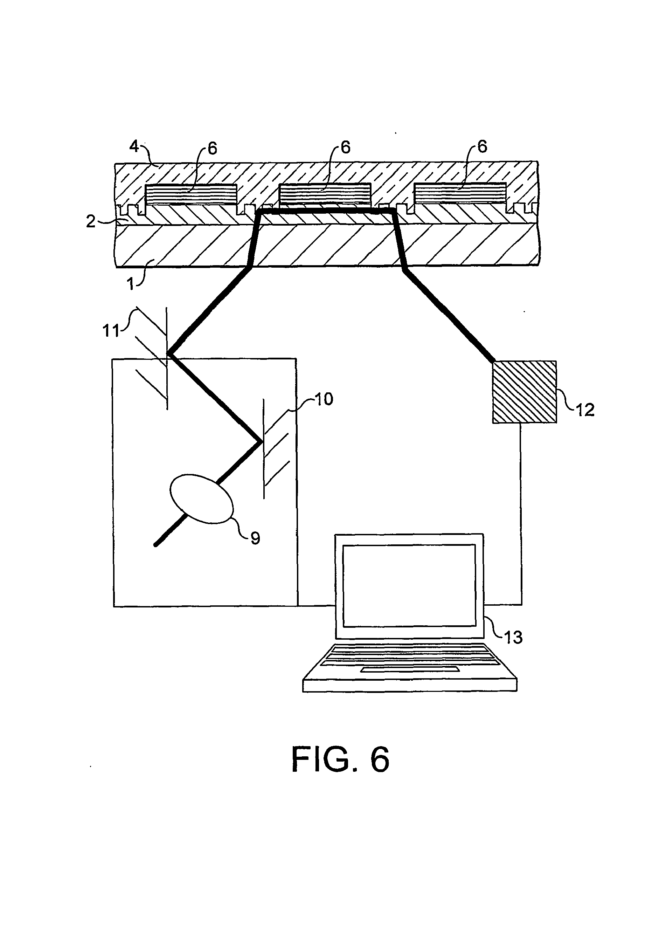

Oligo number 562 was hybridised to the linearised clusters prepared as described above at 500nM. Nucleotide sequences of the template and primers are shown in Figure 6.

Sequencing was carried out using modified nucleotides prepared as described in International patent application WO 2004/018493, and labelled with four different commercially available fluorophores (Molecular Probes Inc.).

A mutant 9°N polymerase enzyme (an exo- variant including the triple mutation L408Y/Y409A/P410V and C223S) was used for the nucleotide incorporation steps.

Enzyme mix (enzymology buffer above plus 50µg/ml of the enzyme, and 1µM each of the four labelled modifies nucleotides) was applied to the clustered templates, typically for 2 min 30s, and heated to 45°C.

Templates were maintained at 45°C for 30 min, cooled to 20°C and washed for 5 min with enzymology buffer, then 5 min with 5x SSC. Templates were then exposed to an imaging buffer of 100mM Tris pH7.0, 30mM NaCl, 50mM sodium ascorbate (freshly dissolved, filtered).

Fluorescent detection is achieved by using the following instrumentation (schematically illustrated in Figure 6) comprising of excitation assembly and detection assembly. The instrument may be assembled using standard optical imaging components.

Excitation assembly: A laser beam is deflected onto the input grating by means of actuated a pair of mirrors 10, 11 as shown on Fig 6. Two mirrors are needed in order to ensure that the grating is excited at a correct place as well as at a correct angle. A laser beam of approximately 1mm diameter is focussed in the direction perpendicular to the grating grooves by a cylindrical lens 9 in order to optimise the coupling efficiency. The laser beam is coupled out at the output grating and the power of it is measured using a photodiode 12. The signal from the photodiode is registered on a control computer 13. As a part of the initial set up, mirrors are scanned in each direction of motion in discreet steps and the signal from the photodiode is monitored at each position. In this initial set-up an attenuated laser beam of approximately 1% of normal power is used. After several iterations of this process, the position of optimum coupling is found and recorded. The full power laser beam may then be used to excite the fluorescence.

Detection assembly: Fluorescent image is obtained at each position along the direction parallel to the direction of reagent flow through the flow cell. If the width of the reagent channel is also 1mm, the field of view will be approximately 1mm² . Nucleic acid colonies on the wave guide are resolved by scanning using a Nikon x20, NA 0.75 microscope objective placed on top of the wave guide, linked to a PentaMax intensified CCD. Templates are scanned in 4 colours (488nm, 532nm, 594 nm and 647nm). Four fluorophores can be measured using four different emission filters. The imaging is done in sequence such that four images are recorded for each area of surface scanned.

Templates are exposed to cycles of enzymology, imaging and cleavage. Each imaging cycle is carried out using the detection instrument-described above. The first enzymatic incorporation step may be done "off-line" to allow the user to set up the instrument for the first scan. Following each incorporation step a wash step may be included to remove unincorporated nucleotides. After imaging of the first (and subsequent) cycle of incorporation the incorporated nucleotide may be cleaved to remove the fluorophore. Second and subsequent nucleotide incorporation steps may then be carried on the instrument.

### TCEP treatment cycle

0.1M Tris pH 7.4 for 390 s
Heat to 45 °C
TCEP (100 mM in 0.1 M Tris pH 7.4) for 390 s
Wait for 30 min, flushing for 20 s every 10 min
Cool to 20 °C
Enzymology buffer for 390 s
5xSSC buffer for 390 s
imaging buffer (100 mM Tris pH 7.0, 30 mM NaCl, 50 mM sodium ascorbate) for 330 s
Scan in 4 colours

### Enzymology cycle

Enzymology buffer for 390 s
Heat to 45 °C
Enzyme mix for 390 s
Wait for 30min, flushing for 20s every 10 min
Cool to 20 °C
Enzymology buffer for 390 s
5xSSC buffer for 390 s
imaging buffer (100 mM Tris pH 7.0, 30 mM NaCl, 50 mM sodium ascorbate) for 330 s, scan 4 colours

## Claims

1. A method of sequencing a template nucleic acid molecule comprising:
immobilizing said template nucleic acid molecule on or proximal to a planar waveguide;
incorporating one or more nucleotides into a strand of nucleic acid complementary to the template nucleic acid, wherein one or more of the incorporated nucleotides comprise a fluorescent label, and determining the identity of the base present in one or more incorporated nucleotide(s) in order to determine the sequence of the template nucleic acid molecule;
wherein the identity of the base present in said nucleotide(s) is determined by detecting fluorescence from the fluorescently labelled nucleotide(s) when exposed to evanescent illumination generated by coupling of light into said planar waveguide.

2. The method according to claim 1 wherein the template nucleic acid is present in an array.

3. The method according to claim 2 wherein the array is a clustered array.

4. The method according to claim 2 wherein the array is a single molecule array.

5. The method according to any one of the preceding claims wherein at least 2 nucleotides are successively incorporated and the identity of the base present in each of the incorporated nucleotides is determined.

6. The method according to claim 5 wherein at least 10 nucleotides are successively incorporated and the identity of the base present in each of the incorporated nucleotides is determined.

7. The method according to claim 6 wherein at least 16 nucleotides are successively incorporated and the identity of the base present in each of the incorporated nucleotides is determined.

8. The method according to claim 7 wherein from 16 to 30 nucleotides are successively incorporated and the identity of the base present in each of the incorporated nucleotides is determined.

9. The method according to any one of the preceding claims wherein the identity of the base present in each of the incorporated nucleotide(s) is determined after each nucleotide incorporation.

10. A device comprising an array of template nucleic acid molecules immobilised on the surface of a planar wave guide wherein the array comprises clusters of template nucleic acid strands located at distinct locations on the array, and wherein the device further comprises nucleotides incorporated into strands complementary to the templates, wherein the nucleotides carry a removable blocking group attached to the 3' position.

11. A device according to claim 10 which further comprises
a top plate held in liquid seal against at least a portion of the surface of the planar wave guide on which the template nucleic acid molecules are immobilised to form a flow cell enclosing at least one reaction channel,
an inlet port and an outlet port to permit flow of liquid through the flow cell when the device is in use, and
an input coupling grating.

12. A device according to claim 11 which further includes an output coupling grating, wherein the input and output coupling gratings are positioned on either side of a reaction channel.

13. A device according to claim 12 which comprises two or more reaction channels, wherein a coupling grating is positioned between each adjacent pair of reaction channels.

14. A device according to claim 13 wherein the reaction channels are each in fluid communication with a common reagent reservoir.

15. Use of a planar wave guide to perform a method of sequencing according to any of claims 1-9.

16. A method of determining identity of the base present in a nucleotide incorporated into a polynucleotide molecule immobilised on a planar wave guide comprising:
incorporating a nucleotide into a strand of nucleic acid complementary to the template nucleic acid, wherein the nucleotide is fluorescently-labelled, and determining the identity of the base present in the incorporated nucleotide by detecting a signal produced by said nucleotide when exposed to an evanescent field generated by coupling of light into said planar waveguide.

## Patentansprüche

1. Verfahren zur Sequenzierung eines Templat-Nukleinsäuremoleküls, wobei das Verfahren die folgenden Schritte aufweist:
Immobilisierung des Templat-Nukleinsäuremoleküls auf oder proximal zu einem planaren Wellenleiter;
Einbau eines oder mehrerer Nukleotide in einen zu der Templat-Nukleinsäure komplementären Nukleinsäurestrang, wobei eines oder mehrere der eingebauten Nukleotide einen Fluoreszenzmarker aufweisen, und Bestimmung der Identität der Base, welche in einem oder mehreren eingebauten Nukleotid(en) vorliegt, um die Sequenz des Templat-Nukleinsäuremoleküls zu bestimmen;
**dadurch gekennzeichnet, dass** die Identität der in dem/den Nukleotid(en) vorliegende Base durch die Erfassung der Fluoreszenz des oder der fluoreszierend markierten Nukleotide bestimmt wird, wenn dieses/diese der durch Lichtkopplung in den planaren Wellenleiter erzeugten evaneszenten Beleuchtung ausgesetzt wird/werden.

2. Verfahren nach Anspruch 1, **dadurch gekennzeichnet, dass** die Templat-Nukleinsäure in einem Array vorliegt.

3. Verfahren nach Anspruch 2, **dadurch gekennzeichnet, dass** das Array ein gebündeltes bzw. Cluster-Array ist.

4. Verfahren nach Anspruch 2, **dadurch gekennzeichnet, dass** das Array ein Einzelmolekül-Array ist.

5. Verfahren nach einem der vorhergehenden Ansprüche, **dadurch gekennzeichnet, dass** mindestens 2 Nukleotide nacheinander eingebaut werden und dass die Identität der in jedem der eingebauten Nukleotide vorliegenden Base bestimmt wird.

6. Verfahren nach Anspruch 5, **dadurch gekennzeichnet, dass** mindestens 10 Nukleotide nacheinander eingebaut werden und dass die Identität der in jedem der eingebauten Nukleotide vorliegenden Base bestimmt wird.

7. Verfahren nach Anspruch 6, **dadurch gekennzeichnet, dass** mindestens 16 Nukleotide nacheinander eingebaut werden und dass die Identität der in jedem der eingebauten Nukleotide vorliegenden Base bestimmt wird.

8. Verfahren nach Anspruch 7, **dadurch gekennzeichnet, dass** 16 bis 30 Nukleotide nacheinander eingebaut werden und dass die Identität der in jedem der eingebauten Nukleotide vorliegenden Base bestimmt wird.

9. Verfahren nach einem der vorhergehenden Ansprüche, **dadurch gekennzeichnet, dass** die Identität der in jedem der eingebauten Nukleotide vorliegenden Base nach jedem Nukleotid-Einbau bestimmt wird.

10. Vorrichtung, welche ein Array von Templat-Nukleinsäuremolekülen aufweist, welche auf der Oberfläche eines planaren Wellenleiters immobilisiert sind, **dadurch gekennzeichnet, dass** das Array Zusammenballungen bzw. Cluster von Templat-Nukleinsäuresträngen aufweist, welche an individuellen Stellen auf dem Array angeordnet sind, und dass die Vorrichtung des Weiteren Nukleotide aufweist, welche in zu den Templaten komplementäre Stränge eingebaut werden, wobei die Nukleotide eine entfernbare Blockierungsgruppe tragen, welche an die 3-Position gebunden ist.

11. Vorrichtung nach Anspruch 10, welche des Weiteren Folgendes aufweist:
eine Deckplatte, welche in Flüssigkeitsverschluss zu mindestens einem Abschnitt der Oberfläche des planaren Wellenleiters gehalten wird, auf welchem die Templat-Nukleinsäuremoleküle zur Bildung einer Durchflusszelle, welche mindestens einen Reaktionsweg umgibt, immobilisiert bzw. bewegungsunfähig gemacht werden;
eine Einlassöffnung und eine Auslassöffnung, um einen Durchfluss der Flüssigkeit durch die Durchflusszelle zu ermöglichen, wenn die Vorrichtung in Betrieb ist; und
ein Eingangskopplungsgitter.

12. Vorrichtung nach Anspruch 11, welche des Weiteren ein Ausgangskopplungsgitter aufweist, **dadurch gekennzeichnet, dass** die Eingangs- und Ausgangskopplungsgitter auf beiden Seiten eines Reaktionswegs positioniert sind.

13. Vorrichtung nach Anspruch 12, welche zwei oder mehr Reaktionswege aufweist, **dadurch gekennzeichnet, dass** ein Kopplungsgitter zwischen jedem benachbarten Paar von Reaktionswegen positioniert ist.

14. Vorrichtung nach Anspruch 13, **dadurch gekennzeichnet, dass** die Reaktionswege jeweils in Fluidverbindung mit einem gemeinsamen Reagenzbehälter sind.

15. Verwendung eines planaren Wellenleiters zur Ausführung eines Sequenzierungsverfahrens nach einem der Ansprüche 1 bis 9.

16. Verfahren zur Identitätsbestimmung der Base, welche in einem Nukleotid vorliegt, das in ein Polynukleotidmolekül eingebaut worden ist, wobei das Polynukleotidmolekül auf einem planaren Wellenleiter immobilisiert wird, wobei das Verfahren den folgenden Schritt aufweist:
Einbau eines Nukleotids in einen zu der Templat-Nukleinsäure komplementären Nukleinsäurestrang, wobei das Nukleotid eine Fluoreszenzmarkierung aufweist, und Bestimmung der Identität der Base, welche in dem eingebauten Nukleotid vorliegt, indem ein von dem Nukleotid erzeugtes Signal erfasst wird, wenn das Nukleotid einem durch Lichtkopplung in den planaren Wellenleiter erzeugten evaneszenten Feld ausgesetzt wird.

## Revendications

1. Procédé de séquençage d'une molécule d'acide nucléique matrice comprenant:
immobiliser ladite molécule d'acide nucléique matrice sur ou à proximité d'un guide d'onde plan;
incorporer un ou plusieurs nucléotides dans un brin de l'acide nucléique complémentaire à l'acide nucléique matrice, où un ou plusieurs des nucléotides incorporés comprennent un marqueur fluorescent, et déterminer l'identité de la base présente dans un ou plusieurs nucléotides incorporés pour déterminer la séquence de la molécule d'acide nucléique matrice;
où l'identité de la base présente dans le ou les nucléotides précités est déterminée en détectant la fluorescence du ou des nucléotides marqués de manière fluorescente lorsqu'ils sont exposés à un éclairage évanescent produit par le couplage de la lumière dans ledit guide d'onde plan.

2. Procédé selon la revendication 1, où l'acide nucléique matrice est présent en une série.

3. Procédé selon la revendication 2, où la série est une série groupée.

4. Procédé selon la revendication 2, où la série est une série de molécules uniques.

5. Procédé selon l'une quelconque des revendications précédentes, où au moins deux nucléotides sont successivement incorporés, et l'identité de la base présente dans chacun des nucléotides incorporés est déterminée.

6. Procédé selon la revendication 5, dans lequel au moins 10 nucléotides sont successivement incorporés, et l'identité de la base présente dans chacun des nucléotides incorporés est déterminée.

7. Procédé selon la revendication 6, où au moins 16 nucléotides sont successivement incorporés, et l'identité de la base présente dans chacun des nucléotides incorporés est déterminée.

8. Procédé selon la revendication 7, dans lequel entre 16 et 30 nucléotides sont successivement incorporés, et l'identité de la base présente dans chacun des nucléotides incorporés est déterminée.

9. Procédé selon l'une quelconque des revendications précédentes, dans lequel l'identité de la base présente dans chacun des nucléotides incorporés est déterminée après chaque incorporation de nucléotide.

10. Dispositif comprenant une série de molécules d'acide nucléique matrices immobilisées sur la surface d'un guide d'onde plan, où la série comprend des groupements de brins d'acide nucléique matrice situés à des emplacements distincts sur la série, et où le dispositif comprend en outre des nucléotides incorporés dans des brins complémentaires aux matrices, où les nucléotides portent un groupe de blocage amovible attaché à la position 3'.

11. Dispositif selon la revendication 10, qui comprend en outre une plaque supérieure retenue d'une manière étanche au liquide contre au moins une portion de la surface du guide d'onde plan, sur laquelle les molécules d'acide nucléique matrice sont immobilisées pour former une cellule d'écoulement renfermant au moins un canal de réaction,
un orifice d'entrée et un orifice de sortie pour permettre l'écoulement du liquide à travers la cellule d'écoulement lorsque le dispositif est en utilisation, et
une grille de couplage d'admission.

12. Dispositif selon la revendication 11, qui comporte en outre une grille de couplage d'évacuation, où les grilles de couplage d'admission et d'évacuation sont positionnées de chaque côté d'un canal de réaction.

13. Dispositif selon la revendication 12, qui comprend deux ou plusieurs canaux de réaction, où une grille de couplage est positionnée entre chaque paire adjacente de canaux de réaction.

14. Dispositif selon la revendication 13, dans lequel les canaux de réaction sont chacun en communication fluidique avec un réservoir de réactif commun.

15. Utilisation d'un guide d'onde plan pour exécuter un procédé de séquençage selon l'une quelconque des revendications 1 à 9.

16. Procédé de détermination de l'identité de la base présente dans un nucléotide incorporé dans une molécule de polynucléotide immobilisée sur un guide d'onde plan comprenant:
incorporer un nucléotide dans un brin d'acide nucléique complémentaire à l'acide nucléique matrice, où le nucléotide est marqué par fluorescence, et déterminer l'identité de la base présente dans le nucléotide incorporé en détectant un signal produit par ledit nucléotide lorsqu'il est exposé à un champ évanescent produit par le couplage de la lumière dans ledit guide d'onde plan.
